# EUROPÄISCHE PATENTSCHRIFT

(11) **EP 1 164 147 B1**
(45) Veröffentlichungstag und Bekanntmachung des Hinweises auf die Patenterteilung: **15.09.2004**
(21) Anmeldenummer: 01112930.1
(22) Anmeldetag: 06.06.2001
(51) Int. Cl.: C08F 8/12, C08F 8/44, A61L 15/60, A61F 13/20

(54) **Verfahren zur kontinuierlichen Herstellung von superabsorbierenden Polymerisaten aus PAN-Emulsionen**
Continuous process for preparing superabsorbent polymers from PAN-emulsions
Procédé en continue pour la préparation de polymères superabsorbants

(30) Priorität: 16.06.2000 DE 10029876
(43) Veröffentlichungstag der Anmeldung: 19.12.2001
(73) Patentinhaber: BAYER AG, 51368 Leverkusen (DE)
(72) Erfinder: Sackmann, Günter, Dr., 51379 Leverkusen (DE); Meyer, Rolf-Volker, Dr., 53804 Much (DE); Schapowalow, Sergej, Dr., 410010 Saratov (RU); Bayburdov, Telman, Dr., 410048 Saratov (RU); Nakonetschny, Jgor, 410065 Saratov (RU)

(56) Entgegenhaltungen:
- EP-A- 0 019 681
- EP-A- 0 670 335
- EP-A- 0 697 416
- EP-A- 0 783 005

## Beschreibung

Die vorliegende Erfindung betrifft ein Verfahren zur kontinuierlichen Herstellung von superabsorbierenden Polymerisaten auf Basis von feinteiligen unvernetzten und vernetzten wässrigen Polyacrylnitril-Emulsionen.

Superabsorbierende Polymerisate sind bekannt und werden hauptsächlich bei der Herstellung von Windeln und Inkontinenzartikeln, aber auch als wasserspeichernde Materialien in der Landwirtschaft sowie zur Ummantelung von Elektrokabeln eingesetzt. In der Regel handelt es sich bei den kommerziell verfügbaren superabsorbierenden Polymerisaten um weitmaschig vernetzte, wasserunlösliche Polymerisate auf Basis von Alkalisalzen der Polyacrylsäure oder von Copolymerisaten aus Acrylsäure und Acrylamid, die durch radikalisch initiierte Copolymerisation von Acrylsäure und polyfunktionellen Monomeren, wie z.B. Divinylbenzol, Ethylenglykoldimethacrylat, Ethylenglykoldiallylether, Butandiolacrylat, Hexandioldimethacrylat, Polyglykoldiacrylat, Trimethylolpropandiacrylat, Allylacrylat, Diallylacrylamid, Triallylamin, Diallylether, Methylenbisacrylamid und N-Methylolacrylamid, erhalten werden. Aufgrund ihrer molekularen Struktur sind solche Polymerisate in der Lage, unter Quellung und Ausbildung von Hydrogelen große Mengen an Flüssigkeiten aufzunehmen und diese auch unter Druck festzuhalten.

In den Patentanmeldungen EP-A-670 335 und EP-A-697 416 werden superabsorbierende Polymerisate mit extrem hohem Quellvermögen und hohen Gelfestigkeiten beschrieben. Diese Produkte erhält man durch alkalische Hydrolyse von Polyacrylnitril (PAN)-Emulsionen bei 50-100°C und Reaktionszeiten von 1 bis 2 Stunden. Bei diesem Verfahren werden nach der Hydrolyse Produkte mit superabsorbierenden Eigenschaften durch Ausfällen mit Lösungsmitteln, wie z.B. aliphatischen Monoalkoholen, als feinteilige Pulver isoliert. Nach dem Filtrieren und Trocknen werden die superabsorbierenden Polymerisate auf die gewünschte Korngröße gemahlen.

Die zur Herstellung der superabsorbierenden Polymerisate benötigten feinteiligen, wässrigen, hochmolekularen, unvernetzten oder vernetzten Polyacrylnitril-Emulsionen werden durch die Homo- und/oder Copolymerisation von Acrylnitril in Gegenwart spezieller anionischer polymerer Emulgatoren erhalten (siehe Patentanmeldungen EP-A-331066 sowie EP-A-590 460). Die Molekulargewichte der nach diesem Verfahren dargestellten unvernetzten Polyacrylnitril-Emulsionen liegen im Bereich von 5·10⁵ bis 1·10⁷ g/Mol, bevorzugt von 2·10⁶ bis 5·10⁶ g/Mol. Die Teilchengrößen der unvernetzten oder vernetzten wässrigen PAN-Emulsionen liegen im Bereich zwischen 100 und 300 nm, bevorzugt zwischen 100 und 200 nm (bestimmt mittels Laser Korrelations- Spektroskopie).

Bei der Hydrolyse solcher PAN-Emulsionen mit wässrigen Lösungen von Alkalihydroxiden entstehen die teilhydrolysierten Homo- und/oder Copolymerisate des Acrylnitrils, worin 30 bis 80 Mol.-% der Nitrilgruppen in Carboxylatgruppen und 20 bis 70 Mol.-% der Nitrilgruppen in Carbonamidgruppen umgewandelt sind und 0 bis 20 Mol.-% der Nitrilgruppen unverändert bleiben.

Aufgrund des bei beginnender Hydrolyse auftretenden Übergangs von der niedrigviskosen PAN-Emulsion in den hochviskosen, wassergequollenen gelartigen Zustand ist eine kontinuierliche Durchführung der beschriebenen Verfahren in konventionellen Rührapparaturen nicht realisierbar. Zur kontinuierlichen Verarbeitung solcher gelartigen Reaktionsgemische werden spezielle Apparaturen benötigt. In der Patentanmeldung EP-A-783 005 ist ein Verfahren zur kontinuierlichen Herstellung von superabsorbierenden Polymerisaten beschrieben, worin man wässrige Emulsionen von vernetzten oder unvernetzten Polyacrylnitril-Homo- und/oder Copolymerisaten in einem für die Durchführung von hochviskosen Reaktionen kontinuierlich arbeitenden, mischenden und knetenden Langzeitreaktor ("List-Reaktor") durch Reaktion mit wässrigen Alkalihydroxidlösungen bei 70 bis 100°C hydrolysiert und aus den sich dabei bildenden hochviskosen Gelen durch kontinuierliche Fällung mit niedrigsiedenden Monoalkoholen (Ethanol, Methanol) das superabsorbierende Polymerisat als leicht filtrierbares Pulver kontinuierlich ausfällt. Nach dem Trocknen und Mahlen auf das gewünschte Korngrößenspektrum liegt dann der fertige Superabsorber vor. Eine solche Apparatur ist im Vergleich zu konventionellen Reaktoren technisch anspruchsvoll. Aufgabe war es daher, ein alternatives deutlich vereinfachtes Verfahren zu finden.

Es wurde nun gefunden, dass superabsorbierende Polymerisate aus PAN-Emulsionen kontinuierlich in konventionellen Reaktoren hergestellt werden können, wenn die Hydrolyse von PAN-Emulsionen in einem wässrig-alkoholischen Medium unter Ammoniak-Abspaltung durchgeführt wird. Der Alkohol dient einerseits als Verdünnungsmittel für die bei der Hydrolyse entstehende hochviskose Zwischenstufe und andererseits als Fällungsmittel.

Im Verlauf der Hydrolyse gehen die feinteiligen Emulsionen auf Basis von Polyacrylnitril in eine leicht rührbare Suspension über. Dadurch wird es möglich, die Hydrolysestufe in Form einer gut rührbaren Suspension in einer Rührkesselkaskade kontinuierlich durchzuführen.

Gegenstand der Erfindung ist ein Verfahren zur Herstellung von superabsorbierenden Polymerisaten, dadurch gekennzeichnet, dass man vernetzte oder unvernetzte feinteilige wässrige Polyacrylnitril-Emulsionen in einer kontinuierlich arbeitenden Rührkesselkaskade durch Reaktion mit Alkalihydroxidlösung in einem wässrig-alkoholischen Medium bei 60 bis 100°C hydrolysiert.

Weiterer Gegenstand der Erfindung ist somit ein Verfahren zur Herstellung von superabsorbierenden Polymerisaten, worin man vernetzte oder unvernetzte wässrige Polyacrylnitril-Emulsionen in einer kontinuierlich arbeitenden Rührkesselkaskade durch Reaktion mit Alkalihydroxidlosung in einem wässrig-alkoholischen Medium bei 60 bis 100°C, bevorzugt 65 bis 80°C, 0,5 bis 3 Stunden, bevorzugt 1 bis 2 Stunden, zur Umsetzung bringt. Dabei liegt das molare Verhältnis der Nitrilgruppen der Ausgangspolymerisate zu den Hydroxylgruppen der Alkalihydroxyde im Bereich von 1:0,5 bis 1:1.

Die Reaktorkaskade zur Herstellung von superabsorbierenden Polymerisaten durch alkalische Hydrolyse von PAN-Emulsionen in einem Wasser-Alkohol-Gemisch besteht aus drei oder mehr Rührkesseln, wobei im ersten Kessel eine Vormischung der Edukte, im zweiten Kessel die Vorhydrolyse- und im dritten Kessel die eigentliche Hydrolysestufe durchgeführt werden.

Ersatzweise kann die Vorhydrolysestufe auch in einem Rohrreaktor vorgenommen werden.

Zur Durchführung der Hydrolysestufe kann man zwei oder mehrere Reaktoren (Rührkessel) verwenden, die alternierend betrieben werden. Dadurch können die nachfolgenden Schritte der Fällung und Neutralisation des superabsorbierenden Polymerisats ohne Unterbrechung direkt anschließend an die Hydrolyse durchgeführt werden.

Der Materialtransport von einem Reaktor zum anderen Reaktor erfolgt nach dem Schwerkraft-Prinzip und benötigt deshalb keine Förderpumpen.

Als Alkohole werden primäre, aliphatische Monoalkohole, bevorzugt Methanol, Ethanol, n-Propanol, n-Butanol eingesetzt. Das Gewichtsverhältnis von Wasser zu Alkohol beträgt dabei 1:0,4 bis 1:2.

Das Gewichtsverhältnis von Polyacrylnitril zu Wasser liegt im Bereich von 1:2 bis 1:6, bevorzugt 1:2 bis 1:5, besonders bevorzugt 1:3 bis 1:4,5. Es wurde gefunden, dass man durch eine Veränderung der Gewichtsverhältnisse von Wasser zu Alkohol sowie durch die Art des Alkohols (Fällungsvermögen) aus unvernetzten PAN-Emulsionen Produkte mit unterschiedlichen Vernetzungsgraden und unterschiedlichen, jedoch steuerbaren anwendungstechnischen Eigenschaften erhalten kann.

Die derart erhältlichen superabsorbierenden Polymerisate weisen ausgezeichnete anwendungstechnische Eigenschaften auf. So erreichen die Produkte, die aus unvernetzten PAN-Emulsionen hergestellt wurden, folgende Quellungsgrade: 150 g/g bis 500 g/g in entionisiertem Wasser und 29 bis 55 g/g in 0,9 %iger NaCl-Lösung.

Werden die auf die oben beschriebene Weise dargestellten Superabsorber noch einer nachträglichen Oberflächenmodifizierung unterworfen, wie sie z.B. in der Patentanmeldung EP-A-936 223 beschrieben ist, dann zeigen die Produkte neben ihrem hohen Quellungsvermögen auch ausgezeichnete Anti-Gelblocking-Eigenschaften, was sich in hohen Absorbency Under Load (AUL)-Werten bei 0,3 psi sowie bei 0,7 psi ausdrückt. Außerdem weisen diese Produkte sehr niedrige wasserlösliche Anteile auf.

Die erfindungsgemäße hergestellten superabsorbierenden Polymerisate werden beispielweise in Hygieneprodukten, wie Babywindeln und Inkontinenzartikeln, als wasserspeichernde Materialien in der Landwirtschaft oder bei der Ummantelung von Elektrokabeln erfindungsgemäß eingesetzt und verwendet.

### Beispiele

### Beispiel 1

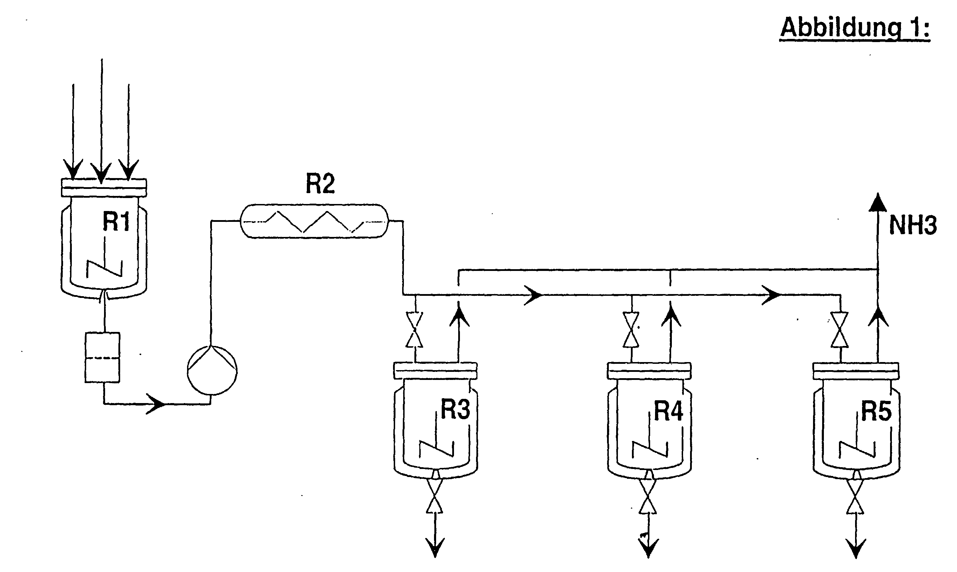

Die zur Herstellung von superabsorbierenden Polymerisaten nach dem erfindungsgemäßen Verfahren verwendete Apparatur (Abbildung 1), besteht aus einer Kaskade von fünf Reaktoren. Reaktor eins (R1 : Rührkessel mit einem freien Volumen 10 l) dient zur Vormischung der Edukte. Reaktor zwei (R2: Rohrreaktor mit einem freien Innenvolumen von ca. 2,0 l, einem Rohrdurchmesser von 1,6 cm sowie einer Gesamtlänge von 10 m) dient zur Aufheizung und Vorhydrolyse von PAN-Emulsionen. In den alternierend betriebenen Reaktoren drei, vier und fünf (R3, R4, R5), bei welchen es sich um Rührkessel mit einem freien Volumen von jeweils 10 l handelt, erfolgen die Hydrolyse unter Ammoniak-Abspaltung, die Nachfällung und die Neutralisation der Superabsorber. Danach werden diese Reaktoren entleert und die wässrig-alkoholischen Suspensionen den weiteren Aufarbeitungsschritten, wie Filtration, Trocknung, Mahlung, Siebung sowie Oberflächenmodifizierung zugeführt.

Der kontinuierliche Transport des Reaktionsgemischs vom zweiten Reaktor (R2) zu den Reaktoren R3, R4 und R5 erfolgt nach dem Schwerkraft-Prinzip mit einer Geschwindigkeit von 3 bis 6 l/h. Lediglich zum Fördern der dünnflüssigen Edukte von R1 nach R2 wird eine Pumpe benötigt.

Für die Hydrolyse wurde eine unvernetzte Polyacrylnitril-Emulsion mit einem Feststoffgehalt von 28,0 Gew.-% und einem [η]-Wert von 8,6 dl/g sowie einer mittleren Teilchengröße von 120 nm eingesetzt.

Mit Hilfe von Pumpen werden die PAN-Emulsion mit einer Strömungsgeschwindigkeit von 2,968 kg/h, Ethanol und eine 46 gew.-%ige wässrige Lösung von NaOH jeweils mit einer Strömungsgeschwindigkeit von 2,091 kg/h in den ersten Rührkessel (R1) zur Vormischung dosiert. Die Vormischung wird bei einer Temperatur von 10 bis 20°C durchgeführt.

Am Beginn der Hydrolyse weist das Ausgangsreaktionsgemisch folgende Zusammensetzung auf:

| | |
|---|---|
| Konzentration an Polyacrylnitril ([PAN]) | 13,40 Gew.-% |
| Konzentration an Natronlauge ([NaOH]) | 7,58 Gew.-% |
| Molverhältnis an PAN zu NaOH | 1:0,75 |
| Konzentration an Ethanol | 33,20 Gew.-% |
| Konzentration an Wasser | 45,46 Gew.-% |
| Gewichtsverhältnis von Wasser zu Ethanol | 1:0,73 |
| Gewichtsverhältnis von PAN zu Wasser | 1:3,39 |

Mit Hilfe einer Pumpe wird das Ausgangsreaktionsgemisch mit einer Strömungsgeschwindigkeit von 6,058 kg/h in den zweiten Reaktor (R2) dosiert, wo es auf eine Temperatur von 65-70°C vorgeheizt und vorhydrolysiert wird. Die Verweilzeit im Reaktor R2 beträgt ca. 20 Minuten.

Der Umsetzungsgrad der Nitrilgruppen nach der Vorhydrolyse beträgt ca. 35 Mol.-% (bestimmt durch IR-Spektroskopie).

Danach fließt das Reaktionsgemisch kontinuierlich zur weiteren Hydrolyse vom zweiten Reaktor (R2) zu den Reaktoren R3, R4, R5, die abwechselnd betrieben werden. Wenn z.B. der Reaktor R3 mit dem Reaktionsgemisch gefüllt ist (ca. 6,0 l), werden die Reaktoren R2 und R4 zusammengeschaltet und der Materialtransport verläuft dann von R2 nach R4 usw. Während sich die Reaktoren R4 und R5 auffüllen, kann das hydrolysierte Reaktionsgemisch im Reaktor R3 weiter aufgearbeitet werden. Durch diese Vorgehensweise ist ein kontinuierlicher Betrieb der beschriebenen Apparatur gewährleistet.

Im Reaktor R3 beträgt die Verweildauer für das Reaktionsgemisch insgesamt 2 Stunden bei einer Hydrolysetemperatur von 76 bis 78°C. Das bei der Hydrolyse entstehende gasförmige Ammoniak wird während der Reaktion aus dem Reaktor über spezielle Austrittsöffnungen und einen Rückflusskühler und anschließende Absorption in Wasser oder durch Tieftemperaturkondensation entfernt.

Nach der Hydrolysereaktion wird das Reaktionsgemisch unter Rühren mit 2,0 kg Ethanol zusätzlich bei einer Temperatur von 45 bis 50°C ca. 10-15 min. ausgefällt und danach wird der Rührer abgestellt. Nach der Ausfällung des Produktes werden 3,88 kg des Wasser-Alkohol-Gemischs aus dem Reaktionsgemischs von oben abgesaugt. Danach werden erneut 2,0 kg eines Wasser-Ethanol-Gemischs in den Reaktor eindosiert.

Nach 10-minütigem Rühren dieser Suspension wird mit 390 g 20 %iger Essigsäure ein pH-Wert zwischen 6 und 7 eingestellt. Nach der Neutralisation wird der Reaktorinhalt über das Bodenventil abgelassen.

In den Reaktoren R4 und R5 werden die Hydrolyse, Ausfällung und Neutralisation unter denselben Bedingungen, wie im Reaktor R3, durchgeführt.

Die erhaltene Superabsorber-Suspension mit einem Feststoffgehalt von 21 Gew.-% wird danach filtriert. Nach dem Filtrieren und Waschen mit einem Wasser-Ethanol-Gemisch wird das erhaltene Rohprodukt mit einem Feststoffgehalt von 36,5 Gew.-% bei einer Temperatur zwischen 70 und 80°C in einem Umluftschrank getrocknet. Danach wird das Produkt auf ein Teilchengrößenspektrum von 100 bis 850 µm gemahlen.

### Bestimmung des Quellungsgrades:

250 mg des zu untersuchenden superabsorbierenden Polymerisats werden in ein 300 ml-Becherglas eingewogen und mit 250 ml bis 300 ml destilliertem Wasser bzw. mit 50 ml einer 0,9 gew.-%igen NaCl-Lösung übergossen und stehen gelassen.

Nach Erreichen des Gleichgewichtsquellungszustandes wird das erhaltene Gel über ein Filtertuch mit einer Maschenweite von 30 µm oder einem Papierfilter abfiltriert und ausgewogen. Der Quellungsgrad errechnet sich dann aus dem Verhältnis Auswaage zu Einwaage in g/g. Jede Bestimmung wird dreimal durchgeführt. Die Messgenauigkeit beträgt ± 5%.

Für das gemäß Beispiel 1 dargestellte Produkt ergibt sich ein Quellungsgrad von 300 g/g in destilliertem Wasser und von 47 g/g in 0,9 %iger NaCl-Lösung.

### pH-Wert-Bestimmung:

Der pH-Wert des gemäß Beispiel 1 erhaltenen Produktes beträgt in 0,9 %iger NaCl-Lösung 6,5.

### Bestimmung des wasserlöslichen Anteils (WLA):

0,5 g des superabsorbierenden Polymerisats werden in 500 ml entionisiertem Wasser aufgequollen und 16 Stunden bei 20°C gerührt. Nach Filtrieren des Gels erhält man aus der Bestimmung des Feststoffgehaltes im Filtrat und im Waschwasser den WLA. Dieser beträgt im Falle des nach Beispiel 1 erhaltenen Produktes 5,9 Gew.-%.

### Beispiele 2 bis 6:

Die Hydrolysebedingungen der nach den Beispielen 2 bis 6 hergestellten Proben sind in Tabelle 1 zusammengefasst. Für diese Beispiele wurde die Hydrolyse der PAN-Emulsion in einem Wasser-Ethanol-Gemisch entsprechend der in Beispiel angegebenen Methode durchgeführt. Dabei wurden die Hydrolysebedingungen (die Konzentration von PAN im Reaktionsgemisch, das Molverhältnis von PAN zu NaOH, das Gewichtsverhältnis von PAN zu Wasser, die Verweilzeit) variiert.

In den vier letzten Spalten der Tabelle 1 sind die Quellungsgrade der hergestellten Produkte in destilliertem Wasser und 0,9 %iger NaCl-Lösung sowie die pH-Werte und die wasserlöslichen Anteile der Produkte bei Teilchengrößen von 100 bis 850 µm aufgeführt. Aus den in Tabelle 1 zusammengefassten Ergebnissen geht eindeutig hervor, dass man durch das erfindungsgemäße Verfahren Produkte mit superabsprbierenden Eigenschaften aus unvernetzten PAN-Emulsionen und unterschiedlichen, jedoch steuerbaren anwendungstechnischen Eigenschaften durch Veränderung der Verhältnisse Wasser/Alkohol sowie PAN/Wasser erhält. Es kann also festgestellt werden, dass durch das erfindungsgemäße Verfahren "maßgeschneiderte Produkte" hergestellt werden können.

Zur Verbesserung der anwendungstechnischen Eigenschaften wurden die nach den Beispielen 1 bis 6 hergestellten superabsorbierenden Polymerisate anschließend einer Oberflächenmodifizierung mit Formaldehyd und Kieselsäure unterzogen, wie es in der Europäischen Patentanmeldung 936 223 beschrieben ist.

In der nachfolgenden Tabelle 2 sind die anwendungstechnischen Eigenschaften der nach Beispielen 1 bis 6 erhaltenen modifizierten Superabsorber zusammengestellt. Gemessen wurden dabei folgende Eigenschaften:
- Absorption nach der Zylinder-Methode wie sie in DE-A 40 15 085 beschrieben ist; dabei wurden alle 2 Sekunden Messwerte on-line genommen. In der Tabelle 2 sind jedoch nur die nach 30 Minuten erhaltenen Werte aufgeführt.
- Retention (gemäß "edana"-Vorschrift 440.0-96)
- AUL (Absorbency Under Load) bei 0,3 psi und 0,7 psi (gemäß "edana"-Vorschrift 440.0-96)

Außerdem sind in der Tabelle 2 die wasserlöslichen Anteile (WLA), die pH-Werte und die Ergebnisse von Rewet-Messungen an den nach den Beispielen 2, 4 und 6 hergestellten superabsorbierenden Polymerisaten angegeben. Alle Untersuchungen wurden mit 0,9 %iger NaCl-Lösung durchgeführt.

Rewet-Messungen dienen zum Nachweis, dass die superabsorbierenden Polymerisate unter praxisnahen Bedingungen, wie z.B. in Babywindeln, Körperflüssigkeiten auch unter Druck festzuhalten vermögen. Dabei werden in einem Zellstoff-Fluff mit der Fläche 13x21 cm 5 g Superabsorber gleichmäßig verteilt und dann mit 70 ml einer 0,9 %igen NaCl-Lösung übergossen. Danach deckt man die Fläche mit einem Stapel Filterpapier ab und belastet 15 sec mit einem Gewicht von 4 kg. Nach Messung der Gewichtszunahme der Filterpapiere wird dieser Vorgang - Aufgabe von 70 ml Flüssigkeit und erneute Belastung mit 4 kg - noch zweimal wiederholt.

Die nach jedem Prüfvorgang gemessene Gewichtszunahme der Filterpapiere stellt ein Maß für die Fähigkeit der Superabsorber dar, Flüssigkeit auch unter großer Belastung festzuhalten.

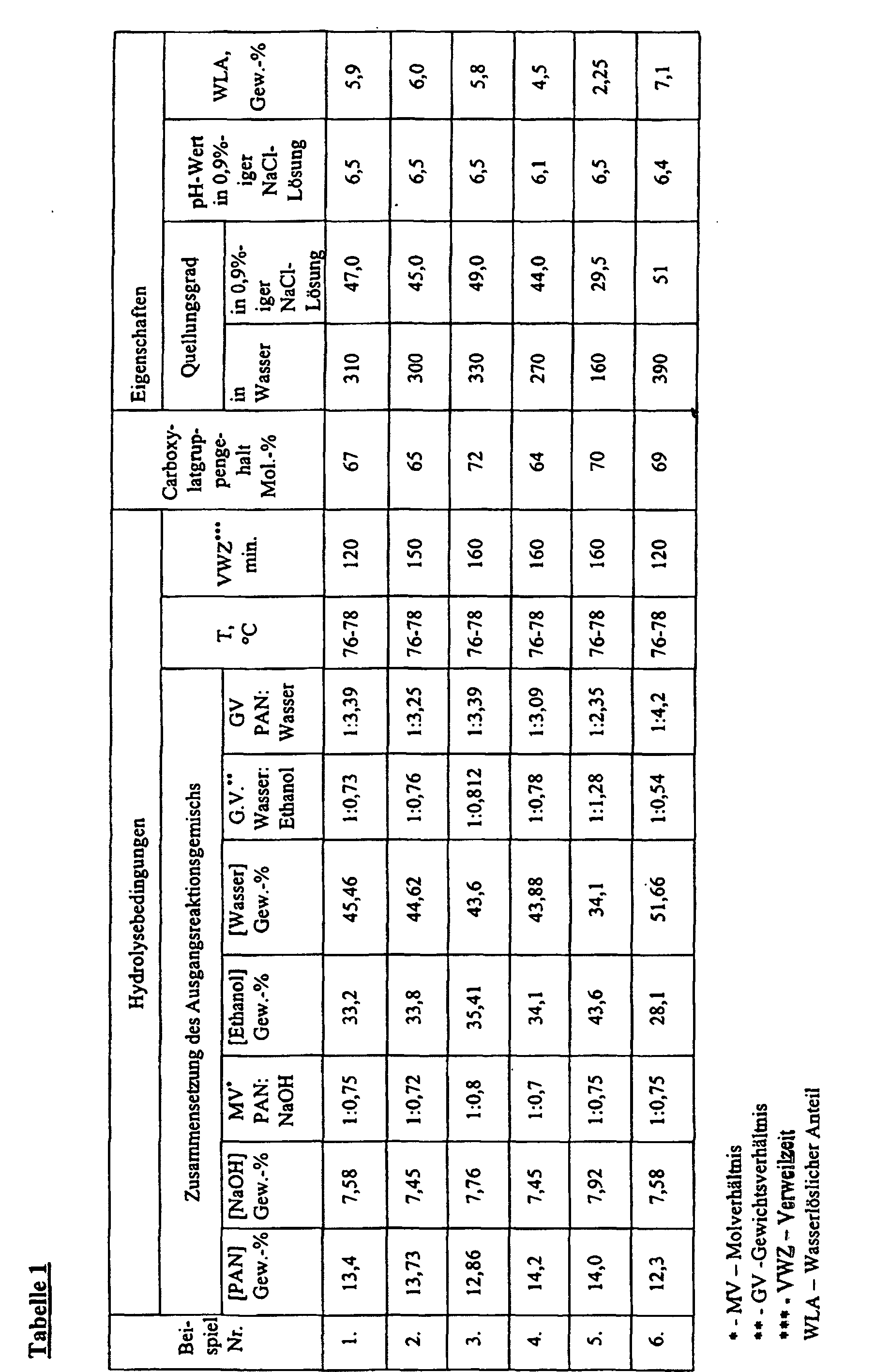

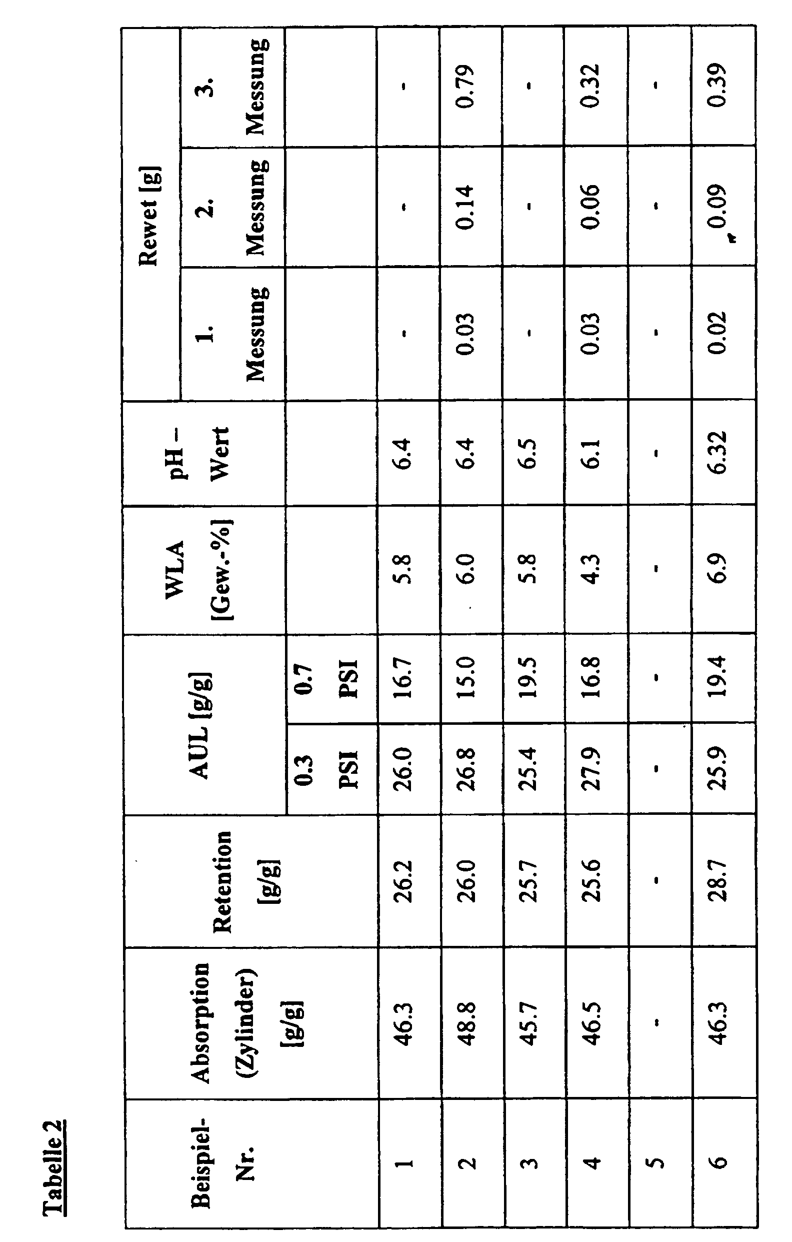

## Patentansprüche

1. Verfahren zur Herstellung von superabsorbierenden Polymerisaten, aus vernetzten oder unvernetzten feinteiligen wässrigen Polyacrylnitril-Emulsionen durch Reaktion mit Alkalihydroxidlösung bei 60 bis 100°C
**dadurch gekennzeichnet, daß** man in einer kontinuierlich arbeitenden Rührkesselkaskade in einem wässrig-alkoholischen Medium hydrolysiert.

2. Verfahren gemäß Anspruch 1, wobei die Zeitdauer der Hydrolyse 0,5 bis 3 Stunden beträgt.

3. Verfahren gemäß einem oder mehrerer der vorangegangenen Ansprüche, wobei das molare Verhältnis der Nitrilgruppen der Ausgangspolymerisate zu den Hydroxylgruppen der Alkalihydroxide zwischen 1:0,5 und 1:1 liegt.

4. Verfahren gemäß einem oder mehreren der vorangegangenen Ansprüche, wobei die Reaktorkaskade zur Herstellung von superabsorbierenden Polymerisaten aus drei oder mehr Rührkesseln besteht, wobei im ersten Kessel eine Vormischung der Edukte im zweiten Kessel die Vorhydrolyse- und im dritten Kessel die eigentliche Hydrolysestufe durchgeführt wird.

5. Verfahren gemäß Anspruch 4, wobei die Vorhydrolysestufe in einem Rohrreaktor vorgenommen wird.

6. Verfahren gemäß einem oder mehreren der vorangegangenen Ansprüche, wobei als Alkohole primäre, aliphatische Monoalkohole eingesetzt werden.

7. Verfahren gemäß einem oder mehreren der vorangegangenen Ansprüche, wobei als Alkohole Methanol, Ethanol, n-Propanol, n-Butanol eingesetzt werden.

8. Verfahren gemäß einem oder mehreren der vorangegangenen Ansprüche, wobei das Gewichtsverhältnis von Wasser zu Alkohol 1:0,4 bis 1:2 beträgt.

9. Verfahren gemäß einem oder mehreren der vorangegangenen Ansprüche, wobei die Hydrolyse der Polyacrylnitril-Emulsionen beim Gewichtsverhältnis von Polyacrylnitril zu Wasser 1:2 bis 1:6 durchgeführt wird.

## Claims

1. A process for producing superabsorbent polymers, from crosslinked or uncrosslinked, fine-particle, aqueous polyacrylonitrile emulsions by reaction with alkali-hydroxide solution at 60 to 100°C, **characterised in that** it is hydrolysed in a continuously operating cascade of stirred tanks in an aqueous alcoholic medium.

2. Process according to Claim 1, wherein the duration of the hydrolysis amounts to 0.5 to 3 hours.

3. Process according to one or more of the preceding claims, wherein the molar ratio of the nitrile groups of the initial polymers to the hydroxyl groups of the alkali hydroxides is between 1:0.5 and 1:1.

4. Process according to one or more of the preceding claims, wherein the cascade of reactors for producing superabsorbent polymers consists of three or more stirred tanks, a premixing of the educts being carried out in the first tank, the prehydrolysis stage being implemented in the second tank, and the actual hydrolysis stage being implemented in the third tank.

5. Process according to Claim 4, wherein the prehydrolysis stage is performed in a tubular reactor.

6. Process according to one or more of the preceding claims, wherein primary, aliphatic monoalcohols are employed by way of alcohols.

7. Process according to one or more of the preceding claims, wherein methanol, ethanol, n-propanol, n-butanol are employed by way of alcohols.

8. Process according to one or more of the preceding claims, wherein the weight ratio of water to alcohol amounts to 1:0.4 to 1:2.

9. Process according to one or more of the preceding claims, wherein the hydrolysis of the polyacrylonitrile emulsions is carried out with a weight ratio of polyacrylonitrile to water of 1:2 to 1:6.

## Revendications

1. Procédé de préparation de polymères superabsorbants à partir d'émulsions aqueuses de polyacrylonitrile finement divisé réticulé ou non réticulé par réaction avec une solution d'hydroxyde alcalin entre 60 et 100°C, **caractérisé en ce qu'**on hydrolyse dans une cascade de récipients agités dans un milieu aqueux alcoolique.

2. Procédé selon la revendication 1, où la durée de l'hydrolyse atteint 0,5 à 3 heures.

3. Procédé selon une ou plusieurs des revendications précédentes, **caractérisé en ce que** le rapport molaire des groupes nitrile du polymère de départ aux groupes hydroxyle de l'hydroxyde alcalin est entre 1 : 0,5 et 1 : 1.

4. Procédé selon une ou plusieurs des revendications précédentes, **caractérisé en ce que** la cascade de réacteurs pour la préparation des polymères superabsorbants est constituée de trois récipients agités ou plus, où dans le premier récipient on effectue un prémélange des produits d'addition, dans le deuxième récipient on effectue la préhydrolyse et dans le troisième récipient on effectue l'étape d'hydrolyse véritable.

5. Procédé selon la revendication 4, dans lequel l'étape de préhydrolyse est effectuée dans un réacteur tubulaire.

6. Procédé selon une ou plusieurs des revendications précédentes, dans lequel on utilise comme alcools des monoalcools primaires, aliphatiques.

7. Procédé selon une ou plusieurs des revendications précédentes, dans lequel on utilise comme alcools le méthanol, l'éthanol, le n-propanol, le n-butanol.

8. Procédé selon une ou plusieurs des revendications précédentes, dans lequel le rapport en poids de l'eau à l'alcool est de 1 : 0,4 à 1 : 2.

9. Procédé selon une ou plusieurs des revendications précédentes, dans lequel on effectue l'hydrolyse des émulsions de polyacrylonitrile selon un rapport en poids du polyacrylonitrile à l'eau de 1 : 2 à 1 : 6.
